# EUROPÄISCHE PATENTSCHRIFT

(11) **EP 2 002 860 B1**
(45) Veröffentlichungstag und Bekanntmachung des Hinweises auf die Patenterteilung: **19.12.2012**
(21) Anmeldenummer: 08007565.8
(22) Anmeldetag: 18.04.2008
(51) Int. Cl.: A61M 39/26, A61M 25/00

(54) **Katheterventil**
Catheter valve
Soupape de cathéter

(30) Priorität: 12.06.2007 DE 102007027623
(43) Veröffentlichungstag der Anmeldung: 17.12.2008
(73) Patentinhaber: Pfleiderer, Klaus, 60433 Frankfurt am Main (DE)
(72) Erfinder: Pfleiderer, Klaus, 60433 Frankfurt am Main (DE)
(74) Vertreter: Erb, Henning

(56) Entgegenhaltungen:
- EP-A- 1 754 501
- CH-A5- 684 165
- DE-A1- 19 718 582
- DE-C1- 19 915 342
- US-A- 5 230 706

## Beschreibung

Die Erfindung betrifft ein Katheterventil mit einem Gehäuse, das einen inneren Hohlraum, eine an einen Ableitungskatheter anschließbare Eintrittsöffnung, eine mit einem Anschlussstück eines Auffangbehälters verbindbare Austrittsöffnung und einen Ventilsitz aufweist, der mit einem in Schließrichtung federnd vorbelasteten, axial in dem Hohlraum geführten Ventilkörper zusammenwirkt, welcher mittels eines an ihm angreifenden, in einem Langloch in der Gehäusewand axial geführten Griffstücks manuell in Öffnungsrichtung verschiebbar ist, wobei die Austrittsöffnung für das Einführen des Anschlussstücks in eine dicht sitzende Anschlussstellung ausgelegt ist, in der es den Ventilkörper vom Ventilsitz weg in Richtung zur Eintrittsöffnung hin in eine Öffnungsstellung zurückgedrängt hat.

Ein derartiges Katheterventil ist aus der DE 10 2005 038 651 A1 bekannt. Die dort beschriebene Konstruktion sieht zwei manschettenförmige Membrandichtungen vor, deren eine die Eintrittsöffnung und das in Strömungsrichtung vordere Ende des hohlen Ventilkörpers dicht umschließt, und deren andere einteilig mit einer elastischen Auskleidung der Austrittsöffnung ausgebildet und mit axialem Zwischenraum zur ersten Membrandichtung dicht sitzend auf dem Ventilkörper befestigt ist. In dem Zwischenraum greift ein am Griffstück angeformter, durch das Langloch in der Gehäusewand radial nach innen vorspringender Ansatz unmittelbar am Ventilkörper an. Eine derartige Ausführung hat zwar im Vergleich zu solchen mit gleitenden Dichtungen, z. B. gemäß EP 0 873 763 B1, den Vorteil zuverlässigerer Abdichtung. Andererseits ist die Konstruktion mit einer vollständigen Eingrenzung des Flüssigkeitsstroms zwischen der Ein- und Austrittsöffnung durch elastische Manschetten innerhalb einer umgebenden Gehäusewand unnötigerweise aufwendig und umständlich zu montieren.

Die CH 684165 A5 beschreibt ebenfalls ein Katheterventil mit einer im wesentlichen vollständigen Eingrenzung des Flüssigkeitsstroms durch eine schlauchförmige Membran im inneren Hohlraum des Ventils. Der Schlauch ist über ein an die Eintrittsöffnung angeschlossenes, biegsames Rohr gezogen, das an seinem hinteren Ende eine im Durchmesser größer als das Rohr bemessene Endwand und davor auf der Seite eines Griffstücks eine Öffnung in der Umfangswand hat. Zum Öffnen des Ventils wird das Griffstück mit Zwischenabstand zur Endwand radial einwärts gegen den Schlauch gedrückt und auf diese Weise das hintere Ende des Rohrs zu der dem Griffstück gegenüberliegenden Seite ausgelenkt. Dadurch hebt auf Seiten des Griffstücks der Rand der Endwand von der Schlauchwand ab, so dass die Flüssigkeit durch die Öffnung in der Umfangswand des Rohrs austreten und an der Endwand vorbei zur Austrittsöffnung strömen kann. Bei dieser Konstruktion führt das axiale Einstecken des Anschlussstücks eines Auffangbehälters nicht unmittelbar und dauerhaft zur Öffnung des Kathederventils. Es ist vielmehr noch zusätzlich das Griffstück radial niederzudrücken und ein daran verschieblich geführter Riegel zu betätigen, um das Ventil geöffnet zu halten.

Der Erfindung liegt deshalb die Aufgabe zugrunde, ein Katheterventil der eingangs genannten Art zur Verfügung zu stellen, das ebenfalls ohne gleitende Dichtungen auskommt, aber einen wesentlich einfacheren Aufbau hat und sich leichter montieren lässt.

Vorstehende Aufgabe wird erfindungsgemäß dadurch gelöst, dass das Langloch durch eine es überdeckende, an der Innenseite der Umfangswand des Gehäuses dicht befestigte, flexible Membran abgedichtet ist, über die das Griffstück an dem Ventilkörper angreift.

Das vorgeschlagene Katheterventil kann mit einem sehr einfach gestalteten, vorzugsweise zylindrischen Gehäuse mit einem einzigen Innenraum auskommen. Die Gehäusewand selbst begrenzt den Flüssigkeitsstrom zwischen Ein- und Austrittsöffnung. Mit einer fest angebrachten Membrandichtung ist lediglich das Langloch abgedichtet, durch das sich das Griffstück erstreckt. Diese Dichtung lässt sich ohne weiteres so gestalten, dass sie den durch das Langloch radial nach innen bis zum Ventilkörper reichenden Ansatz des Griffstücks umgibt und seine zum Verschieben des Ventilkörpers notwendige axiale Bewegung zulässt. Es hat sich überraschend gezeigt, dass die zwischen dem Griffstück und dem Ventilkörper hindurchgeführte Dichtungsmembran, die dort auch der federnden Vorbelastung des Ventilkörpers ausgesetzt ist, weder funktional störend noch selbst störanfällig ist.

Die das Langloch abdichtende Membran ist in der bevorzugten praktischen Ausführung der Erfindung eine sich im axialen Bereich des Langlochs um den Umfang des inneren Hohlraums erstreckende Manschette, die axial neben dem Langloch jeweils über den Umfang der Gehäusewand mit dichtem Sitz an dieser festgelegt ist. In weiterer bevorzugter Ausgestaltung der Erfindung ist diese Manschette im axialen und Umfangsbereich des Langlochs mit einer flexiblen, radial einwärts bis zum Ventilkörper reichenden Tasche und im übrigen hohlzylindrisch mit einem zum Durchmesser des inneren Hohlraums passenden Außendurchmesser geformt. Die axialen Endbereiche der Manschette können durch radial innen anliegende Stabilisierungsringe in Anlage an der Innenseite der Umfangswand des Gehäuses gehalten sein. Alternativ oder zusätzlich kann man die axialen Endbereiche der Membran durch Kleben an der Gehäusewand dicht befestigen.

Ebenso wie bei der eingangs zitierten bekannten Konstruktion ist für die Gestaltung der Austrittsöffnung und des Ventilsitzes zweckmäßigerweise ein Ring aus gummielastischem Material vorgesehen. Er braucht aber nicht mehr einstückig mit einer Dichtmembran ausgebildet zu sein, sondern kann mit einem kragenförmigen axialen Ansatz auf der Außenseite der Umfangswand des Gehäuses sitzen und dort durch einen durch Bördelung axial fixierten Überwurfring gehalten sein.

Der Ventilkörper ist vorzugsweise auf einem rohrförmigen, axial in den innern Hohlraum ragenden Mündungsstutzen der Eintrittsöffnung axial geführt, auf dem auch eine zwischen dem Ventilkörper und der vorderen Endwand des innern Hohlraums einspannte Schraubenfeder sitzt. Alternativ besteht z. B. die Möglichkeit, dass der Ventilkörper mit wenigstens einer flanschartigen, mit Durchlässen versehenen Erweiterung an der Umfangswand des Gehäuses geführt und mit der Kraft einer an der vorderen Endwand des inneren Hohlraums abgestützten Schraubenfeder beaufschlagt ist.

Nachstehend werden Ausführungsbeispiele der Erfindung anhand der beiliegenden Zeichnung näher erläutert. Es zeigen:
- Fig. 1: einen vereinfachten Längsschnitt einer ersten Ausführung des erfindungsgemäßen Katheterventils und
- Fig. 2: einen entsprechenden Längsschnitt durch ein zweites Ausführungsbeispiel des vorgeschlagenen Katheterventils.

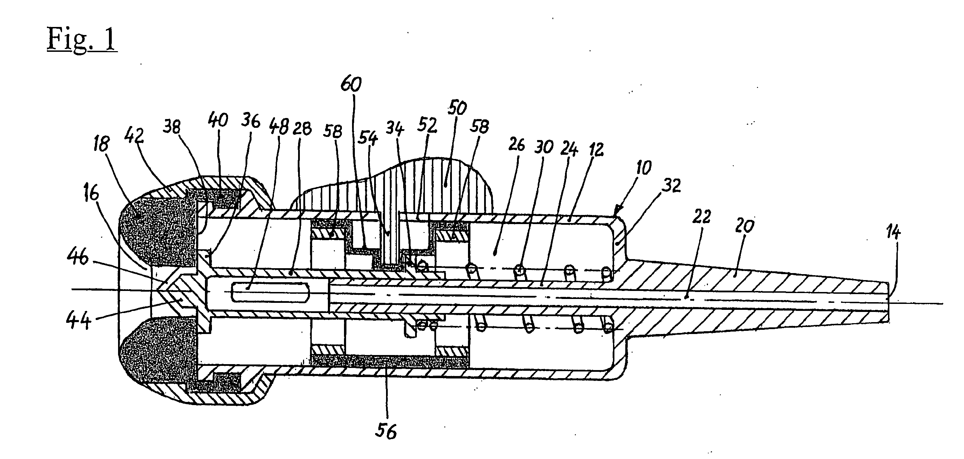

Das in Fig. 1 dargestellte Katheterventil hat ein Gehäuse 10 mit einer im wesentlichen zylindrischen Umfangswand 12, einer Eintrittsöffnung 14, an die ein nicht dargestellter Ableitungskatheter angeschlossen werden kann, und einer Austrittsöffnung 16, die einen sich von außen nach innen verengenden lichten Querschnitt hat, durch einen fest mit dem Gehäuse 10 verbundenen, gummielastischen Ring 18 begrenzt ist und mit einem nicht gezeigten Anschlussstück eines ebenfalls nicht gezeigten Auffangbehälters zu verbinden ist. Die Eintrittsöffnung 14 befindet sich am freien Ende eines im wesentlichen konischen, vorspringenden Zapfens 20, auf den das Ende eines Ableitungskateters aufgeschoben werden kann. An die Eintrittsöffnung 14 schließt sich ein Eintrittskanal 22 an, der sich durch den Zapfen 20 und weiter durch einen rohrförmigen Mündungsstutzen 24 erstreckt, der axial in den Innenraum 26 des Gehäuses 10 ragt. Auf dem Stutzen 24 ist ein hohler Ventilkörper 28 mit einem geschlossenen vorderen Ende axial verschieblich geführt. Außerdem sitz auf dem Stutzen 24 eine als Druckfeder wirkende Schraubenfeder 30, die axial zwischen der vorderen Endwand 32 des Gehäuses 10 und einem äußeren Flansch 34 nahe dem hinteren Ende des Ventilkörpers 28 eingespannt ist. Die Feder 30 stellt eine Vorbelastung des Ventilkörpers 28 dar. Sie hat das Bestreben, einen an dessen hinterem Ende angebrachten Flansch 36 gegen die innenseitige Endfläche 38 des Rings 18 aus gummielastischem Material anzudrücken und in dichter Anlage zu halten. Diese Innenfläche des Rings 18 stellt einen Ventilsitz eines zusammen mit dem Flansch 36 des Ventilkörpers 28 gebildeten Ventils dar.

Der Ring 18 ist mit einem kragenförmigen Ansatz 40 geformt, der in eine Ringnut in der äußeren Umfangsfläche der Gehäusewand 12 einschnappbar ist. Der Ring 18 wird durch einen Überwurfring 42 dauerhaft gehalten, der durch Umbördeln seines axial inneren Endes einen äußerlich verdickten, axialen Endbereich, in dem die genannte Ringnut eingeformt ist, umgreift.

Das hinterste Ende 44 des Ventilskörpers 28, welches sich hinter dem Flansch 36 befindet und im geschlossenen Zustand des Ventils gemäß Fig. 1 in die vom Ring 18 aus gummielastischem Material umgrenzte Austrittsöffnung 16 ragt, ist mit äußeren Rippen 46 geformt. Wenn ein Anschlussstück eines nicht gezeigten Auffangbehälters in die Austrittsöffnung 16 eingeführt wird, stößt es gegen die Rippen 46 und drückt den Ventilkörper 28 gegen die Kraft der Feder 30 in Richtung zur Eintrittsöffnung hin. Dadurch hebt der Flansch 36 des Ventilkörpers 28 von der Sitzfläche 38 des Ventils ab, sodass Urin oder eine andere abzuleitende Flüssigkeit durch die Austrittsöffnung 16 ausströmen und sich in dem an die Austrittsöffnung 16 angeschlossen, nicht gezeigten Auffangbehälter sammeln kann. Der Weg der Flüssigkeit führt dabei durch die Eintrittsöffnung 14, den Eintrittskanal 22, den hohlen, nur am vordern Ende geschlossenen Ventilkörper 28, ein oder mehrere radiale Öffnungen 48 im rohrförmigen Teil des Ventilkörpers 28 und den inneren Hohlraum 26 radial außen um den Flansch 36 herum durch den Öffnungsspalt zwischen dem Flansch 36 und dem Ventilsitz 38.

Das Katheterventil kann auch manuell mittels eines Griffstücks 50 in die Öffnungsstellung gebracht werden. Zu diesem Zweck ist das Griffstück 50 mit einem durch ein Langloch 52 in der Umfangswand 12 des Gehäuses 10 hindurch radial nach innen ragenden Ansatz 54 geformt. Wie in Fig. 1 gezeigt, erstreckt sich dieser Ansatz 54 bis über den Außendurchmesser des vordern Flanschs 34 am Ventilkörper 28 hinaus radial nach innen, so dass er bei Bewegung des Griffstücks 50 in Richtung zur Eintrittsöffnung 14 hin gegen die Kraft der Feder 30 über den Flansch 34 den Ventilkörper 28 mitnimmt.

Damit durch das Langloch 52 keine Flüssigkeit aus dem Gehäuse 10 austritt, ist an der Innenseite der Umfangswand 12 des Gehäuses 10 eine manschettenförmige Membrandichtung 56 befestigt. Sie hat hauptsächlich die Form eines Schlauchabschnitts, dessen Außendurchmesser zum Innendurchmesser der Umfangswand 12 des Gehäuses 10 passt. In den beiden axialen Endbereichen der manschettenförmigen Membrandichtung 56 sorgen radial innen anliegende Stabilisierungsringe 58 für eine sichere, dichte Anlage der manschettenförmigen Dichtung entlang des gesamten Umfangs. Mit oder ohne Verwendung von Stabilisierungsringen können die axialen Randbereiche der Dichtung auch mit der Innenseite der Gehäusewand 12 verklebt werden. Dadurch wird das von der Membrandichtung überdeckte Langloch 52 zuverlässig abgedichtet.

Damit die notwendige axiale Beweglichkeit des Griffstücks 50 trotz der Membrandichtung 56 gewährleistet ist, ist in deren im wesentlichen hohlzylindrische Form im mittleren axialen Bereich, also zwischen den Stabilisierungsringen 58 an wenigstens einer Stelle des Umfangs eine sich nach radial einwärts erstreckende Tasche 60 eingeformt. Im Ausführungsbeispiel bildet die Tasche 60 im Längsschnitt eine stufenförmige Einsenkung. Die Höhe der Stufen ist so gewählt, dass der zentrale, tiefste Teil der Tasche 60 dem Querschnitt des radial nach innen reichenden Ansatzes 54 am Griffstück 50 angepasst ist. Die Zwischenstufe der Tasche befindet sich unmittelbar außerhalb des Umfangs des Flanschs 34, so dass die Beweglichkeit des Ventilkörpers 28 nicht gefährdet ist. Die Tatsache, dass das Griffstück 50 nicht unmittelbar mit seinem radialen Ansatz 54 gegen den Flansch 34 des Ventilkörpers 28 drückt, sondern über die Tasche 60 der Membrandichtung 56 macht sich bei der Handhabung des Katheterventils praktisch nicht bemerkbar.

Es versteht sich, dass die Erfindung auch funktioniert, wenn die Membrandichtung 56 nicht manschettenförmig ist, sondern nur in enger Nachbarschaft zum Langloch 52 um dieses herum an der Innenwand des Gehäuses 10 angeklebt ist. Außerdem besteht die Möglichkeit, eine manschettenförmige Membrandichtung im axial mittleren Bereich mit einer umlaufenden Ringrille zu formen, die denselben Längsschnitt hat wie die Tasche 60.

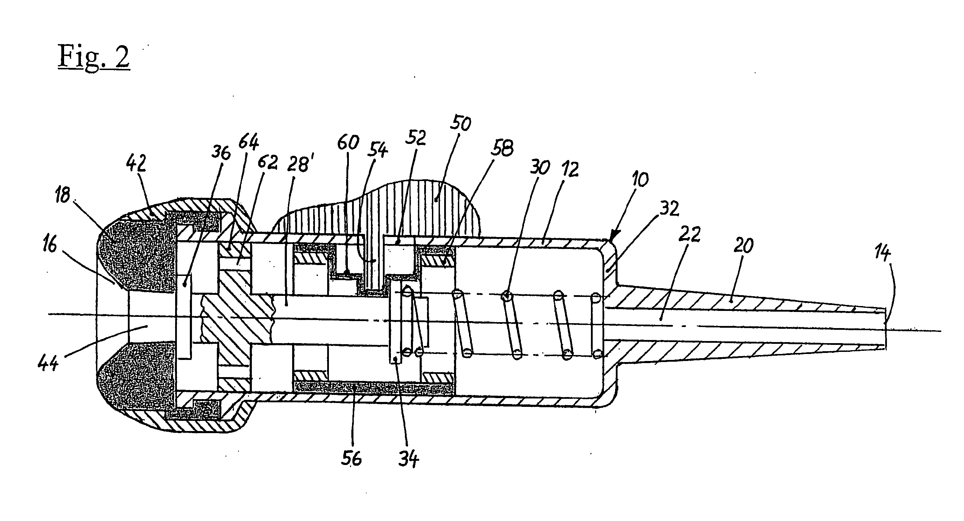

Das Ausführungsbeispiel nach Fig. 2, bei dem identische oder entsprechende Teile mit denselben Bezugszeichen wie in Fig. 1 versehen sind, unterscheidet sich nur dadurch von dem Ausführungsbeispiel nach Fig. 1, dass der rohrförmige Mündungsstutzen 24 fehlt, der Ventilkörper 28' nahe seinem hinteren Ende über einen mit Durchlässen 62 versehenen Flansch 64 an der Innenseite der Umfangswand 12 des Gehäuses 10 in axialer Richtung geführt ist. Wegen der Durchlässe 62 im Flansch 64 braucht der Ventilkörper 28 nicht hohl ausgebildet und mit einer radialen Öffnung 48 versehen zu sein. Seine Länge ist in einem weiten Rahmen frei wählbar. So könnte z. B. auch ein Ventilkörper 28, der zusätzlich zum Flansch 64 einen weiteren derartigen Flansch an seinem einlassseitigen Ende aufweist und beidseits der manschettenförmigen Membrandichtung 56 an der Gehäusewand 12 geführt ist, Verwendung finden.

Wie Fig. 2 zeigt, macht auch die Feder 30 nicht unbedingt einen langen Mündungsstutzen 24 des Einlasskanals 22 notwendig. Es genügt, wenn die Enden der Feder 30 einerseits auf dem Ventilkörper 28 und andererseits an der vorderen Endwand 32 des inneren Hohlraums 26 des Gehäuses 10 festgelegt sind.

Vorzugsweise bestehen alle festen Teile der vorstehend beschriebenen Ausführungsbeispiele des Katheterventils aus Kunststoff, mit Ausnahme der Schraubenfeder 30. Auch die Stabilisierungsringe 58 könnten aus Stahl bestehen und ggf. schräg geschlitzte Federringe sein, welche die manschettenförmige Membrandichtung 56 durch Federkraft gegen die Gehäusewand 12 andrücken.

Zur Montage der gezeigten Ausführungsbeispiele des Katheterventils wird zunächst die Membrandichtung 56 eingesetzt. Dann folgen die Feder 30 und der Ventilkörper 28. Danach wird das Griffstück 50, 54 in das Langloch 52 eingerastet und dabei gleichzeitig in die Tasche 60 der Membrandichtung 56 eingeführt. Schließlich wird der gummielastische Ring 18 mit seinem kragenförmigen Ansatz 40 auf dem hinteren Ende des Gehäuses 10 eingerastet und fixiert, indem der Überwurfring 42 von hinten aufgeschoben und an seiner Vorderkante einwärts umgebördelt wird.

## Patentansprüche

1. Katheterventil mit einem Gehäuse (10), das einen inneren Hohlraum (26), eine an einen Ableitungskatheter anschließbare Eintrittsöffnung (14), eine mit einem Anschlussstück eines Auffangbehälters verbindbare Austrittsöffnung (16) und einen Ventilsitz (38) aufweist, der mit einem in Schließrichtung federnd vorbelasteten, axial in dem Hohlraum (26) geführten Ventilkörper (28, 28') zusammenwirkt, welcher mittels eines an ihm angreifenden, in einem Langloch (52) in der Gehäusewand (12) axial geführten Griffstücks (50, 54) manuell in Öffnungsrichtung verschiebbar ist, wobei die Austrittsöffnung (16) für das Einführen des Anschlussstücks in eine dicht sitzende Anschlussstellung ausgelegt ist, in der es den Ventilkörper (28, 28') vom Ventilsitz (38) weg in Richtung zur Eintrittsöffnung (14) hin in eine Öffnungsstellung zurückgedrängt hat, **dadurch gekennzeichnet, dass** das Langloch (52) durch eine es überdeckende, an der Innenseite der Umfangswand (12) des Gehäuses (10) dicht befestigte, flexible Membran (56, 60) abgedichtet ist, über die das Griffstück (50, 54) an dem Ventilkörper (28, 28') angreift.

2. Katheterventil nach Anspruch 1, **dadurch gekennzeichnet, dass** die Membran (56) eine sich im axialen Bereich des Langlochs (52) um den Umfang des inneren Hohlraums (26) erstreckende Manschette ist.

3. Katheterventil nach Anspruch 2, **dadurch gekennzeichnet, dass** die Manschette (56) im axialen und Umfangsbereich des Langlochs (52) mit einer flexiblen, radial einwärts bis zum Ventilkörper (28, 28') reichenden Tasche (60)
und im übrigen hohlzylindrisch mit einem zum Durchmesser des inneren Hohlraums (26) passenden Außendurchmesser geformt ist.

4. Katheterventil nach Anspruch 3, **dadurch gekennzeichnet, dass** die axialen Endbereiche der Manschette (56) durch radial innen anliegende Stabilisierungsringe (58) in Anlage an der Innenseite der Umfangswand (12) des Gehäuses (10) gehalten ist.

5. Katheterventil nach einem der Ansprüche 1 bis 4, **dadurch gekennzeichnet, dass** die Membran (56) durch Kleben an der Gehäusewand (12) dicht befestigt ist.

6. Katheterventil nach einem der Ansprüche 1 bis 5, **dadurch gekennzeichnet, dass** die Austrittsöffnung (16) und der Ventilsitz (38) durch einen Ring (18) aus gummielastischem Material gebildet sind, der mit einem kragenförmigen axialen Ansatz (40) formschlüssig auf der Außenseite der Umfangswand (12) des Gehäuses (10) sitzt und dort durch einen durch Bördelung axial fixierten Überwurfring (42) gehalten ist.

7. Katheterventil nach einem der Ansprüche 1 bis 6, **dadurch gekennzeichnet, dass** der Ventilkörper (28) auf einem rohrförmigen, in den inneren Hohlraum (26) ragenden Mündungsstutzen (24) der Eintrittsöffnung (14, 22) axial geführt ist, auf dem auch eine zwischen dem Ventilkörper (28) und der vorderen Endwand des inneren Hohlraums (26) eingespannte Schraubenfeder (30) sitzt.

8. Katheterventil nach einem der Ansprüche 1 bis 6, **dadurch gekennzeichnet, dass** der Ventilkörper (28') mit wenigstens einer flanschartigen, mit Durchlässen (62) versehenen Erweiterung (64) an der Umfangswand (12) des Gehäuses (10) geführt und mit der Kraft einer an der vorderen Endwand des innern Hohlraums (26) abgestützten Schraubenfeder (30) beaufschlagt ist.

## Claims

1. A catheter valve with a housing (10) which has an inner cavity (26), an inlet opening (14) which can be connected to a drain catheter, an outlet opening (16), which can be connected to a connector of a receptacle, and a valve seat (38) which co-operates with a valve body (28,28') which is spring-loaded in the closing direction and guided axially in the cavity (26), and which by means of a handle (50,54), which engages thereon and is guided axially in a slot (52) in the housing wall (12), can be displaced manually in the opening direction, wherein the outlet opening (16) is designed for the insertion of the connector in a fluidtightly seated connecting position in which it has forced the valve body (28,28') away from the valve seat (38) towards the inlet opening (14) into an open position, **characterised in that** the slot (52) is sealed by a flexible membrane (56,60) which overlaps it and which is fluidtightly fastened on the inside of the peripheral wall (12) of the housing (10) and by way of which the handle (50,54) engages on the valve body (28,28').

2. A catheter valve according to Claim 1, **characterised in that** the membrane (56) is a sleeve extending in the axial region of the slot (52) around the periphery of the inner cavity (26).

3. A catheter valve according to Claim 2, **characterised in that** in the axial and peripheral region of the slot (52) the sleeve (56) is formed with a flexible pouch (60) extending radially inwards up to the valve body (28,28') and, in addition, is of hollow cylindrical form with an outer diameter matching the diameter of the inner cavity (26).

4. A catheter valve according to Claim 3, **characterised in that** the axial end regions of the sleeve (56) is/are retained by radially inwardly adjoining stabilising rings (58) abutting against the inside of the peripheral wall (12) of the housing (10).

5. A catheter valve according to any one of Claims 1 to 4, **characterised in that** the membrane (56) is fluidtightly fastened by being adhered to the housing wall (12).

6. A catheter valve according to any one of Claims 1 to 5, **characterised in that** the outlet opening (16) and the valve seat (38) are formed by a ring (18) of rubber-elastic material which is seated with a collar-like axial projection (40) in a form-locking manner on the outer side of the peripheral wall (12) of the housing (10) and is retained there by a cap ring (42) which is axially located by flanging.

7. A catheter valve according to any one of Claims 1 to 6, **characterised in that** the valve body (28) is guided axially on a tube-shaped orifice connection (24) of the inlet opening (14,22), which projects into the inner cavity (26) and on which there is also seated a helical spring (30) clamped between the valve body (28) and the front end wall of the inner cavity (26).

8. A catheter valve according to any one of Claims 1 to 6, **characterised in that** the valve body (28') is guided with at least one flange-type widening (64), provided with passages (62, on the peripheral wall (12) of the housing (12) and is acted upon by the force of a helical spring (30) supported against the front end wall of the inner cavity (26).

## Revendications

1. °) Soupape de cathéter comportant un boitier (10) comprenant une cavité interne (26), une ouverture d'entrée (14) pouvant être reliée à un cathéter de dérivation, une ouverture de sortie (16) pouvant être reliée à un élément de connexion d'un récipient de réception et un siège de soupape (38), qui coopère avec un corps de soupape (28, 28') guidé axialement dans la cavité (26), élastiquement précontraint dans la direction de fermeture, et pouvant être déplacée manuellement en translation dans la direction d'ouverture au moyen d'une manette (50, 54) venant en prise avec lui et guidée axialement dans un perçage longitudinal (52) dans la paroi du boitier (12), l'ouverture de sortie (16) étant conçue pour permettre l'introduction de l'élément de connexion dans une position de liaison hermétique, dans laquelle il repousse le corps de soupape (28, 28') du siège de soupape (38) vers l'ouverture d'entrée (14), dans une position d'ouverture,
**caractérisée en ce que**
le perçage longitudinal (52) est rendu étanche par une membrane flexible (56, 60) de recouvrement, fixée hermétiquement sur la face interne de la paroi périphérique (12) du boitier (10), par l'intermédiaire de laquelle la manette (50, 54) vient en prise avec le corps de soupape (28, 28').

2. °) Soupape de cathéter conforme à la revendication 1,
**caractérisée en ce que**
la membrane (56) est une manchette s'étendant dans la zone axiale du perçage longitudinal (52) autour de la périphérie de la cavité interne (26).

3. °) Soupape de cathéter conforme à la revendication 2,
**caractérisée en ce que**
la manchette (56) est équipée, dans la zone axiale et périphérique du perçage longitudinal (52) d'une poche flexible (60) s'étendant radialement vers l'intérieur jusqu'au corps de soupape (28, 28') et essentiellement réalisée sous la forme d'un cylindre creux ayant un diamètre externe correspondant au diamètre de la cavité interne (26).

4. °) Soupape de cathéter conforme à la revendication 3,
**caractérisée en ce que**
la zone d'extrémité axiale de la manchette (56) est maintenue en appui contre la face interne de la paroi périphérique (12) du boitier (10) par des bagues de stabilisation (58) s'appliquant radialement vers l'intérieur.

5. °) Soupape de cathéter conforme à l'une des revendications 1 à 4, **caractérisée en ce que**
la membrane (56) est fixée hermétiquement par collage sur la paroi du boitier (12).

6. °) Soupape de cathéter conforme à l'une des revendications 1 à 5, **caractérisée en ce que**
l'ouverture de sortie (16) et le siège de soupape (38) sont formés par une bague (18) en un matériau ayant l'élasticité du caoutchouc qui s'appuie par une liaison par la forme par une saillie axiale (40) en forme de collerette, contre la face externe de la paroi périphérique (12) du boitier (10) et y est maintenue par une bague chapeau (42) fixée axialement par bridage.

7. °) Soupape de cathéter conforme à l'une des revendications 1 à 6, **caractérisée en ce que**
le corps de soupape (28) est guidé axialement sur un embout d'appui tubulaire (24) de l'ouverture d'entrée (14, 22) qui pénètre dans la cavité interne (26), et sur lequel est monté un ressort à boudin (30) précontraint entre le corps de soupape (28) et la paroi d'extrémité avant de la cavité interne (26).

8. °) Soupape de cathéter conforme à l'une des revendications 1 à 6, **caractérisée en ce que**
le corps de soupape (28') est guidé sur la paroi périphérique (12) du boitier (10) par au moins une extension (64) en forme de collerette équipée de passages (62), et est sollicité par la force d'un ressort à boudin (30) s'appuyant contre la paroi d'extrémité avant de la cavité interne (26).
